# NEUE EUROPÄISCHE PATENTSCHRIFT

(11) **EP 0 387 782 B2**
(45) Veröffentlichungstag und Bekanntmachung des Hinweises auf die Entscheidung über den Einspruch: **06.11.1996**
(45) Hinweis auf die Patenterteilung: 04.01.1995
(21) Anmeldenummer: 90104718.3
(22) Anmeldetag: 13.03.1990
(51) Int. Cl.: A61K 31/44, A61K 9/18, A61K 9/22

(54) **Feste pharmazeutische Präparate und Verfahren zu ihrer Herstellung**
Solid pharmaceutical preparations and process for their manufacture
Préparations pharmaceutiques solides et leurs procédés de fabrication

(30) Priorität: 14.03.1989 HU 121589
(43) Veröffentlichungstag der Anmeldung: 19.09.1990
(73) Patentinhaber: EGIS GYOGYSZERGYAR, Budapest X (HU)
(72) Erfinder: Erdos, Sándor, Dr., Budapest (HU); Kenderfi, Jozsef, Dr., H-1124 Budapest (HU); Bárczay, Erzsébet, H-1072 Budapest (HU); Hegedüs, Aranka née Szima, H-1144 Budapest (HU); Krisztián, Mária, H-1161 Budapest (HU); Mándi, Attila, Dr., H-1022 Budapest (HU); Tajthy, Eva, née Juhász, H-1025 Budapest (HU); Tömpe, Péter, Dr., H-1116 Budapest (HU); Csörgo, Margit, Dr., H-1174 Budapest (HU); Fekete, Márton, Dr., H-1027 Budapest (HU); Görgényi, Frigyes, Dr., H-1115 Budapest (HU); Torma, Zoltán, Dr., H-1126 Budapest (HU)
(74) Vertreter: Beetz & Partner Patentanwälte

(56) Entgegenhaltungen:
- EP-A- 0 142 561
- EP-A- 0 232 155
- EP-A- 0 267 386
- EP-A- 0 274 176
- WO-A-86/01717
- US-A- 4 369 172
- Römps Chemie-Lexikon, 3. Auflage, S. 1546
- Sugimoto et al., Chem. Pharm. Bull. 30, 4479 (1982)

## Beschreibung

Die Erfindung betrifft feste pharmazeutische Präparate mit einer regulierten Wirkstoffabgabe, die als Wirkstoff Nifedipin (4-(2-Nitrophenyl)-2,6-dimethyl-3,5-dimethoxycarbonyl-1,4-dihydropyridin) enthalten, sowie ein Verfahren zu ihrer Herstellung

Es ist bekannt, daß Nifedipin ein wertvoller Calciumantagonist ist und zur Behandlung von Bluthochdruck und Coronarkrankheiten verwendet werden kann (DE-A-1 620 827). Die therapeutische Anwendung und Konfektionierung des Nifedipins wird durch die schlechte Wasserlöslichkeit und seine hohe Lichtempfindlichkeit erschwert.

Nifedipin wird in zwei grundsätzlich verschiedenen pharmazeutischen Präparateformen in der Praxis verwendet.

Der erste Typ wird im Falle akuter Anfälle eingesetzt. Um den Anfall so rasch wie möglich zu lösen, ist hier eine rasche Freisetzung des Wirkstoffs erforderlich, und zwar zusammen mit einer raschen Erzielung eines hohen Blutspiegels.

Der andere Typ dient zur Verhütung eines Coronaranfalls und zur Behandlung des Bluthochdrucks. In diesem Falle ist eine rasche Erzielung eines hohen Blutspiegels überhaupt nicht notwendig, ja wegen des Auftretens von Nebenwirkungen sogar unerwünscht. Die therapeutische Wirkung dauert von 2 bis 3 Stunden bis zu 8 bis 10 Stunden. Hierzu werden pharmazeutische Präparate mit einer verhältnismäßig raschen und mehr oder weniger verzögerten Wirkstoffabgabe eingesetzt.

Nach DE 3 033 919 A wird die geringe Löslichkeit des Nifedipins in Wasser und im Verdauungssaft durch Erhöhung der spezifischen Oberfläche in einem solchen Maße verbessert, daß der Wirkstoff aus den Präparaten innerhalb einer therapeutisch annehmbaren Zeitdauer freigesetzt wird. Nach dieser Druckschrift wird Nifedipin in einer Stiftmühle zu einem Pulver mit einer spezifischen Oberfläche von 0,5 bis 6 m²/g gemahlen und danach unter Anwendung von bekannten Hilfsstoffen und Trägern in feste pharmazeutische Präparate übergeführt. Es ergibt sich jedoch aus der Natur des Zerkleinerungsvorganges, daß die Teilchengröße des Wirkstoffs nur zwischen bestimmten Grenzen eingestellt werden kann und die Teilchengrößenverteilung des Wirkstoffs auch innerhalb eines engen Teilchengrößenintervalls je nach Ansatz wesentliche Abweichungen zeigen kann. Ein Teilchengrößenintervall von etwa 1 bis 10 µm (dies entspricht einer spezifischen Oberfläche von 0,5 bis 6 m²/g) kann im Maximum der Teilchengrößenverteilungsfunktion sogar eine zwei- bis dreifache Abweichung hervorrufen, die wiederum die Wirkstoffabgabe beeinflussen kann.

Nach HU 193 287 werden nifedipinhaltige zweiphasige feste pharmazeutische Präparate so hergestellt, daß Nifedipin und ein Kopräzipitatbildner (vorzugsweise Polyvinylpyrrolidon) in einem organischen Lösungsmittel gelöst werden und das nach Entfernen des Lösungsmittels erhaltene Kopräzipitat mit, auf 1 Gew.-Teil des Nifedipingehalts bezogen, 1 bis 5 Gew.-Teilen kristallinem Nifedipin mit einer mittleren Teilchengröße von 10 bis 1 µm und einer spezifischen Oberfläche von 1,0 bis 6,0 m²/g vermischt wird. Das Nifedipin ist jedoch in nicht einheitlichen Granalien enthalten. Bei dem Vermischen der beiden verschiedenen Granalien kann während der Tablettierung bzw. Herstellung der Kapseln eine Entmischung stattfinden.

Aus EP-A-142 561 waren Nifedipinpräparate mit regulierter Wirkstoffabgabe bekannt, bei denen eine rasche Wirkstofffreisetzung durch das Vorliegen von kristallinem Nifedipin in sehr kleiner Teilchengröße von ≦ 5 µm erzielt wird. Eine verzögerte Nifedipinabgabe wird andererseits nach diesem Stand der Technik durch Beschichten der feinen Nifedipinkristalle mit einer nichttoxischen, schlecht wasserlöslichen Substanz bewirkt.

Aus EP-A-232 155 waren ferner Arzneimittelzusammensetzungen mit modifizierter Wirkstofffreisetzung bekannt, die unter anderen Nifedipin als Wirkstoff enthalten können und bei denen der Wirkstoff an einem inaktiven, vernetzten Polymer als Modifizierungsmittel sorbiert ist. Nach diesem Stand der Technik wird eine verzögerte Wirkstofffreisetzung durch Formulierung des Wirkstoffs mit einer Kombination eines wasserlöslichen mit einem wasserunlöslichen Polymer erzielt. Es ist angegeben, daß ein sehr wenig wasserlöslicher Wirkstoff, wie Nifedipin, in den entsprechenden Zusammensetzungen in amorpher Form vorliegt.

Wirkstoffe mit einer calciumantagonistischen Wirkung werden in immer größeren Mengen verwendet. Die oben erwähnten therapeutischen Indikationen erfordern entsprechend pharmazeutische Präparate mit immer präziser und genauer definierten Wirkstoffabgabemerkmalen. Es besteht ein Bedürfnis nach einem ganzen Bereich von pharmazeutischen Präparaten, und zwar von Präparaten, die durch verhältnismäßig rasche Wirkung rasch einen hohen Blutspiegel ergeben, bis zu Präparaten mit verzögerter Wirkung. Unter Berücksichtigung der schlechten Löslichkeit des Nifedipins in Wasser und im Verdauungssaft und der hohen Lichtempfindlichtkeit dieses Wirkstoffs konnten die obigen Ziele mit Hilfe der bekannten Methoden nicht im erwünschten Ausmaß erreicht werden.

Aufgabe der vorliegenden Erfindung ist es, den obigen Forderungen entsprechende Nifedipin-Präparate sowie einfache Verfahren zu ihrer Herstellung anzugeben.

Die Aufgabe wird gemäß den unabhängigen Ansprüchen gelöst. Die abhängigen Ansprüche betreffen vorteilhafte Ausführungsformen.

Die erfindungsgemäßen, Nifedipin (4-(2-Nitrophenyl)-2,6-dimethyl-3,5-dimethoxycarbonyl-1,4-dihydropyridin) enthaltenden festen pharmazeutischen Präparate mit regulierter Wirkstoffabgabe enthalten mindestens ein pharmazeutisch inaktives Hydrophilisierungsmittel, das eine rasche Freisetzung des Nifedipins bewirkt, und mindestens ein pharmazeutisch inaktives Retardierungsmittel, welches eine verzögerte Freisetzung des Nifedipins bewirkt,
und sind erhalten durch
(A) Aufbringen des Nifedipins sowie des Hydrophilisierungsmittels und des Retardierungsmittels in Lösung in einem organischen Lösungsmittel auf einen inerten Träger,
(B) Trocknen des behandelten Trägers,
(C) Vermischen mit üblichen Hilfsstoffen
   und
(D) Tablettieren oder Abfüllen in Kapseln;

Sie sind dadurch gekennzeichnet, daß
- das Nifedipin in kristalliner Form vorliegt,
- das Hydrophilisierungsmittel unter Hydroxypropylcellulose, Polyethylenglycolen und Polyoxyethylenstearat ausgewählt ist,
- das Retardierungsmittel unter Eudragiten® und Polyvinylbutyral ausgewählt ist und
- die Präparate dadurch erhältlich sind, daß Schritt A wie folgt durchgeführt wird:
   (A1) Aufbringen einer Lösung des Nifedipins, des Hydrophilisierungsmittels und des Retardierungsmittels auf den Träger oder
   (A2) Aufbringen einer Lösung des Nifedipins, des Retardierungsmittels sowie einer Teilmenge des Hydrophilisierungsmittels auf den Träger und abschließendes Aufbringen der Restmenge des Hydrophilisierungsmittels
      oder
   (A3) Aufbringen einer Lösung des Nifedipins und des Retardierungsmittels auf den Träger und anschließendes Aufbringen des Hydrophilisierungsmittels,
wobei die aufgebrachten Lösungen aur 1 Gew.-Teil Nifedipin 0,05 bis 1,5 Gew.-Teile Retardierungsmittel und insgesamt 0,1 bis 1,5 Gew.-Teile Hydrophilisierungsmittel enthalten.

Das erfindungsgemäße Verfahren zur Herstellung der Nifedipin-Präparate weist entsprechend die obigen Merkmale und Schnitte auf.

Unter pharmazeutischen Präparaten mit einer verhältnismäßig raschen Wirkstoffabgabe werden im Rahmen der vorliegenden Erfindung feste Arzneimittelformen verstanden, aus welchen 50 % des Wirkstoffgehalts in einer Zeitdauer von höchstens einer Stunde freigesetzt werden (nach der in USP beschriebenen Methode bestimmt) und welche bei Verabreichung einer Tablette oder einer Kapsel zwecks Herabsetzung der Nebenwirkungen eine Plasmakonzentration ergeben, die den wert von 80 ng/ml nicht einmal für eine vorübergehende kurze Zeitdauer überschreitet.

Unter pharmazeutischen Präparaten mit einer verzögerten Wirkstoffabgabe werden andererseits feste Arzneimittelformen verstanden, bei denen zur Freisetzung von 50 % des

Wirkstoffgehalts mehr als eine Stunde notwendig ist.

Die Erfindung beruht auf der Erkenntnis, daß die aus der schlechten Löslichkeit des Nifedipins und der Verschiedenheit der Teilchenstruktur der verwendeten kristallinen Substanz stammenden Unsicherheitsfaktoren und die damit verbundenen Probleme dadurch behoben werden können, daß man Nifedipin in eine Lösung - d.h. eine molekulare Dispersion - bringt und mit einem Hydrophilisierungsmittel und einem Retardierungsmittel zusammen oder teilweise getrennt auf einen festen Träger aufbringt. Die Geschwindigkeit und die Art und Weise der Nifedipinabgabe kann durch das Mengenverhältnis und die Auswahl der beiden Hilfsstoffstypen bzw. das. Mengenverhältnis zwischen Nifedipin einerseits und den beiden Hilfsstoffen andererseits sowie auch durch die Methode des Aufbringens eingestellt werden. Es kann auf diese Weise nicht nur die Geschwindigkeit der Wirkstoffabgabe, sondern auch der Verlauf der Freisetzungskurve modifiziert werden.

Das wesentliche Merkmal des erfindungsgemäßen Verfahrens besteht darin, daß man das Nifedipin auf den Träger nicht allein aufbringt, sondern die Nifedipinlösung immer ein Hydrophilisierungsmittel bzw. ein Retardierungsmittel (oder mindestens einen Teil einer der obigen Komponenten) enthält.

Falls man das Hydrophilisierungsmittel oder einen Teil davon allein (als solches) auf den Träger aufbringt, geschieht dies immer in der letzten Stufe. Dies bezieht sich auch auf den Fall, wenn die Lösung des Hydrophilisierungsmittels oder eines Teiles davon auch einen Teil des Nifedipins enthält.

Das Maß der Wirkstoffabgabe ist von der weiteren Verarbeitung des so erhaltenen Produkts (Verpressen zu Tabletten, Überziehen der Tabletten, Füllung in Kapseln) vollständig unabhängig. Die Zerkleinerung und das Mahlen des Wirkstoffs erübrigen sich und dementsprechend spielt die Teilchenstruktur und Teilchengrößenverteilung des Ausgangsstoffs keine Rolle. Die daraus folgenden statistischen Fluktuationen werden also vermieden. Nach dem erfindungsgemäßen Verfahren wird Nifedipin in Form eines einzigen Granulats verarbeitet, es findet also keine Entmischung statt, und dementsprechend wird keine Abweichung des Blutspiegels bzw. der Aktivität für Präparate ein- und desselben Ansatzes beobachtet.

Ein wesentliches Merkmal der vorliegenden Erfindung besteht darin, daß das Endprodukt das Nifedipin in kristalliner - d.h. nicht-amorpher - Form enthält. Wegen der Anwesenheit von weiteren, zusammen mit dem Nifedipin aufgelösten Komponenten sind diese jedoch keine gut definierten Nifedipinkristalle, sondern liegen eher als Beschichtung auf dem Träger vor.

Nach dem erfindungsgemäßen Verfahren werden als Hydrophilisierungsmittel Polyethylenglycole, Hydroxypropylcellulose oder verschiedene grenzflächenaktive Mittel (z.B. Macrogolstearat) verwendet. Die Hydroxypropylcellulose hat sich als besonders vorteilhaft erwiesen.

Als Retardierungsmittel können vorteilhaft Ethylcellulose, Polyvinylacetat, Polyvinylbutyral oder verschiedene Eudragit® Typen (vorzugsweise Eudragit® RS) eingesetzt werden. Zu diesem Zweck kann man besonders vorteilhaft Polyvinylbutyral verwenden.

Nach einer besonders vorteilhaften Ausführungsform des erfindunsgemäßen Verfahrens werden als Hydrophilisierungsmittel Hydroxypropylcellulose und als Retardierungsmittel Polyvinylbutyral verwendet.

Es ist weiterhin zu berücksichtigen, daß die Wirksamkeit der Hydrophilisierungsmittel bzw. Retardierungsmittel verschieden stark ist. Zum Erreichen einer bestimmten Retardierung ist z.B. eine kleinere Menge an Polyvinylbutyral als von Eudragit® RS ausreichend. Die hydrophilisierende Wirkung von Hydroxypropylcellulose ist höher als die der Polyethylenglycole. Dementsprechend kann das Verhältnis der beiden Hilfsstoffkomponenten, in Abhängigkeit vom ihrer Art und der gewünschten Geschwindigkeit der Wirkstoffabgabe, innerhalb weiter Grenzen variieren.

Es werden 0,1 bis 1,5 Gew.-Teile Hydrophilisierungsmittel und 0,05 bis 1,5 Gew.-Teile Retardierungsmittel, auf 1 Gew.-Teil Nifedipin bezogen, verwendet.

Nach einer Ausführungsform des erfindungsgemäßen Verfahrens werden feste pharmazeutische Präparate mit einer verhältnismäßig raschen Wirkstoffabgabe so hergestellt, daß man 0,3 bis 1,5 Gew.-Teile Hydrophilisierungsmittel und 0,05 bis 0.2 Gew.-Teile Retardierungsmittel, auf 1 Gew.-Teil Nifedipin bezogen, verwendet.

Nach einer anderen Ausführungsform des erfindungsgemäßen Verfahrens werden feste pharmazeutische Präparate mit einer verzögerten Wirkstoffabgabe so hergestellt. daß man 0.10 bis 0.30 Gew.-Teile Hydrophilisierungsmittel und 0.2 bis 1,5 Gew.-Teile Retardierungsmittel, auf 1 Gew.-Teil Nifedipin bezogen, verwendet.

Nach einer besonders vorteilhaften Ausführungsform des erfindungsgemäßen Verfahrens werden feste pharmazeutische Präparate mit einer verhältnismäßig raschen Wirkstoffabgabe so hergestellt. daß man 0.4 Gew.-Teile Hydroxypropylcellulose und 0.07 Gew.-Teile Polyvinylbutyral, auf 1 Gew.-Teil Nifedipin bezogen. verwendet.

Nach einer anderen vorteilhaften Ausführungsform des erfindungsgemäßen Verfahrens werden feste pharmazeutische Präparate mit einer verzögerten Wirkstoffabgabe so hergestellt, daß man 0.1 bis 0.2 Gew.-Teile und vorzugsweise 0.1 Gew.-Teil Hydrophilisierungsmittel und 0.3 bis 0.5 Gew.-Teile und vorzugsweise 0.45 Gew.-Teile Retardierungsmittel, auf 1 Gew.-Teil Nifedipin berechnet, verwendet.

Nach dem erfindungsgemäßen Verfahren werden zur Auflösung des Nifedipins, des Hydrophilisierungsmittels und des Retardierungsmittels vorteilhaft niedere Alkanole, insbesondere Ethanol oder Isopropanol, oder Aceton verwendet. Man kann besonders vorteilhaft mit Ethanol hergestellte Lösungen einsetzen.

Nach dem erfindungsgemäßen Verfahren können das Nifedipin, das Hydrophilisierungsmittel und das Retardierungsmittel in einem organischen Lösungsmittel, vorteilhaft in Ethanol, separat gelöst werden. Die erhaltenen Lösungen werden miteinander in beliebigem Verhältnis vermischt und nacheinander oder nach vollständigem Vermischen auf einmal auf den festen Träger aufgebracht. Man kann so verfahren, daß man das Nifedipin zusammen mit dem oder den Hydrophilisierungsmitteln in Ethanol löst und die so erhaltene Lösung mit der separat hergestellten Lösung des Retardierungsmittels vermischt. Man kann auch so verfahren, daß man das Hydrophilisierungsmittel und das Retardierungsmittel zusammen auflöst und die so erhaltene Lösung mit der Nifedipinlösung vermischt. In bestimmten Fällen kann man vorteilhaft so verfahren, daß man das Hydrophilisierungsmittel oder einen Teil davon nicht mit der Lösung des Nifedipins und des Retardierungsmittels vermischt, sondern es nach Besprühung des Trägers mit diesen Lösungen nachträglich auf den Träger aufbringt. Es soll betont werden, daß die Nifedipinlösung nie allein als solche auf den Träger aufgebracht wird.

Die hergestellten Lösungen werden mit Hilfe von Preßluft nach einem bekannten Wirbelschichtverfahren auf den festen pulverförmigen Träger aufgebracht. Das erfindungsgemäße Verfahren kann auch mittels eines üblichen Knetverfahrens durchgeführt werden. In diesem Falle kann das Aufbringen in Abhängigkeit von der Konzentration der Lösung auch absatzweise mit intermittierenden Trocknungsphasen vollzogen werden. Die Wirbelschicht granulierung hat sich als besonders vorteilhaft erwiesen.

Als Ergebnis des obigen Vorgangs entstehen aus dem feinpulverförmigen Träger körnige Granalien; dies vereinfacht die darauffolgende Verarbeitung (Tablettierung, Füllung in Kapseln) wesentlich.

Als inerte Träger können beliebige pharmazeutisch geeignete Hilfsstoffe oder deren Gemische eingesetzt werden. Man kann vorteilhaft eine Mischung von mikrokristalliner Cellulose und Milchzucker, gegebenenfalls zusammen mit Zerfallsmitteln, wie z.B. Croscarmellose, verwenden.

Die aus dem festen Träger und der aufgebrachten Lösung hergestellten Granalien werden auf bekannte Weise nach beliebigen Verfahren getrocknet und gesiebt. Nach Zugabe von weiteren üblichen Hilfsstoffen, z.B. Talk, Magnesiumstearat, Zerfallsmittel, wie Croscarmellose usw., wird das Gemisch in an sich bekannter Weise zu vorteilhaft konvexen Tabletten gepreßt oder in Kapseln gefüllt.

Wegen der hohen Lichtempfindlichkeit des Nifedipins werden die Tabletten mit einem Überzug versehen. Es kann ein üblicher Überzug auf Zuckerbasis oder ein Filmüberzug aufgebracht werden. Um einen besseren Lichtschutz zu gewährleisten, enthält der Überzug vorteilhaft einen oder mehrere geeignete Farbstoffe oder ein oder mehrere Pigmente. Rote und/oder orangefarbige Farbstoffe, rote und/oder orangefarbige Aluminiumpigmente und/oder Eisenoxidpigmente haben sich als besonders vorteilhaft erwiesen, vorzugsweise in Gegenwart von Titandioxid. Die Kapselwand kann vorzugsweise auch die obigen Lichtschutzmittel enthalten.

Der Vorteil des erfindungsgemäßen Verfahrens besteht darin, daß es auch im Betrieb bequem durchführbar ist und die Wirkstoffabgabe durch geeignete Veränderungen durch Variation der Mengen und Mengenverhältnisse des Hydrophilisierungsmittels einerseits und des Retardierungsmittels andererseits einfach und wirksam eingestellt werden kann.

Das erfindungsgemäße Verfahren ist zur Herstellung von festen pharmazeutischen Präparaten mit einer sowohl verhältnismäßig raschen als auch mit einer verzögerten Wirkstoffabgabe geeignet. Die Freisetzung des Wirkstoffs ist vom pH-Wert des Mediums, z.B. von der Aufenthaltszeit im Magen, unabhängig. Bei den erfindungsgemäßen Präparaten hängt die Wirkstoffabgabe auch nicht von der Teilchenstruktur des als Ausgangsstoff eingesetzten Wirkstoffs ab; die mehrere Unsicherheitsfaktoren hervorrufenden Zerkleinerungs- und Mahlungsvorgänge erübrigen sich. Das Nifedipin ist in einem einheitlichen Granulat enthalten; demzufolge tritt bei der Tablettierung bzw. Herstellung der Kapseln keine Entmischung auf.

Die Erfindung betrifft ferner Nifedipin-Kombinationspräparate, die mindestens ein Präparat mit verhältnismäßig rascher Wirkstoffabgabe sowie mindestens ein Präparat mit verzögerter Wirkstoffabgabe enthalten. Die beiden Präparatarten können dabei z.B. zusammen in Kapseln oder in Tabletten bzw. Dragees verpreßt vorliegen.

Nach einer vorteilhaften Ausführungsform sind die Nifedipin-KombinationspräparateTabletten bzw. Dragees, die eine äußere Schicht aus einem Präparat mit verhältnismäßig rascher Wirkstoffabgabe und einen Kern aus einem Präparat mit verzögerter Wirkstoffabgabe aufweisen.

Weitere Einzelheiten des erfindungsgemäßen Verfahrens sind den nachstehenden Beispielen zu entnehmen.

### Beispiel 1

30 g Nifedipin werden in 240 g Ethanol gelöst. Zu der so erhaltenen Lösung werden eine Lösung von 6 g Hydroxypropylcellulose (Klucal LF) in 50 g Ethanol und danach 240 g 12,5 %ige Eudragit® RS-Lösung (12,5 % Eudragit® RS, 35 % Aceton, 52,5 % Isopropanol, C.A. Reg. No. 33 434-24-1) zugegeben. Die so erhaltene Lösung wird mit Hilfe eines Wirbelschicht verfahrens auf ein gemisch von 184,5 g mikrokristalliner Cellulose, 84 g Milchzucker und 1,5 g Crospovidon (USP XXI/N.F. XVI) gesprüht. Es wird durch ein Sieb von 0,9 mm gesiebt und getrocknet. Nach Zugabe von 18 g Crospovidon, 9 g Talk und 1,5 g Magnesiumstearat wird das Gemisch zu Tabletten verpreßt und mit einem Überzug versehen oder in Kapseln gefüllt. Nach der in USP XXI oder PhHg VII beschriebenen Methode (drehender Korb; 150 Umdrehungen/Minute; in 900 ml einer 0,1 N Salzsäinrelösung) wird die Hälfte des Wirkstoffs innerhalb von 3 Stunden abgegeben (Halbwertszeit T₅₀ = 3 Stunden).

### Beispiel 2

35 g Nifedipin werden in 280 g Ethanol gelöst. Zu der Lösung werden 280 g einer 12,5 %igen Eudragit® RS-Lösung zugegeben, worauf die gebildete Lösung auf ein gemisch von 210 g Cellulose und 70 g Milchzucker gesprüht wird. Es wird danach mit einer Lösung von 5,25 g Hydroxypropylcellulose und 100 g Ethanol besprüht, getrocknet und gesiebt. Nach Zugabe von 21 g Crospovidon, 7 g Talk und 1,75 Magnesiumstearat wird das Gemisch zu Tabletten verpreßt. Die Halbwertszeit T₅₀ beträgt 4 Stunden.

### Beispiel 3

Zu einer Lösung von 40 g Nifedipin und 320 g Ethanol wird eine Lösung von 16 g Polyvinylbutyral (Mowital B) und 270 g Ethanol zugegeben. Die so erhaltene Lösung wird auf ein Gemisch von 180,8 g Cellulose und 32 g Milchzucker gesprüht. Dieser Träger wird mit einer Lösung von 6 g Hydroxypropylcellulose und 115 g Ethanol besprüht, getrocknet und gesiebt. Nach Zugabe von 40 g Croscarmellose (USP XXI/N.F. XVI; vernetzte Natriumcarboxymethylcellulose, Zerfallsmittel), 4 g Talk und 1,2 g Magnesiumstearat wird das Gemisch zu Tabletten verpreßt und nötigenfalls mit einem Überzug versehen. Halbwertszeit T₅₀ = 5 Stunden.

### Beispiel 4

50 g Nifedipin werden in 400 g Ethanol gelöst. Die so erhaltene Lösung wird mit einer Lösung von 25 g Polyvinylbutyral und 300 g Ethanol vermischt. Die erhaltene Lösung wird auf ein gemisch von 232,5 g Cellulose und 50 g Milchzucker gesprüht, worauf der Träger mit einer Lösung von 10 g Hydroxypropylcellulose und 190 g Ethanol besprüht wird. Nach Trocknen und Sieben werden 25 g Croscarmellose und 7,5 g Talk zugegeben. Das Gemisch wird zu Tabletten verpreßt und mit einem Überzug versehen. Halbwertszeit T₅₀ = 6 Stunden.

### Beispiel 5

30 g Nifedipin, 30 g Polyethylenglycol 6000 (Macrogol 6000) und 3 g Polyoxy-40-stearat (Macrogolstearat) werden in 240 g Ethanol gelöst. Die Lösung wird mit einer Lösung von 21 g Polyvinylbutyral und 200 g Ethanol vermischt. Die so erhaltene Lösung wird auf ein gemisch von 195 g Cellulose, 60 g Milchzucker und 18 g Crospovidon gesprüht, getrocknet und gesiebt. Nach Zugabe von 5,9 g Crospovidon, 1,5 g Magnesiumstearat und 0,6 g kolloidalem Siliciumdioxid wird das Gemisch zu Tabletten verpreßt und mit einem Überzug versehen. Halbwertszeit T₅₀ = 4,5 Stunden.

### Beispiel 6

Man verfährt wie in Beispiel 4 mit dem Unterschied, daß man die ethanolische Lösung der Hydroxypropylcellulose in zwei Teile teilt, 50 % dieser Lösung vor dem Besprühen des Trägers zu der nifedipin- und polyvinylbutyralhaltigen ethanolischen Lösung zugibt und den zweiten Teil davon nachträglich durch Sprühen auf den Träger aufbringt.

### Beispiel 7

8 kg Nifedipin werden in 64 kg Ethanol warm gelöst. Zu der Lösung wird eine Lösung von 3,2 kg Hydroxypropylcellulose, 0,56 kg Polyvinylbutyral und 32 kg Ethanol zugegeben. Die erhaltene Lösung wird in einem Wirbelschicht granulierer auf ein gemisch von 36,8 kg mikrokristalliner Cellulose, 12 kg Milchzucker und 2,4 kg Croscarmellose gesprüht. Nach Beendigung des Besprühens werden die Granalien durch ein etwa 0,7 mm weites Sieb gesiebt und getrocknet. Nach Zugabe von 8 kg Croscarmellose, 0,8 kg Talk und 0,24 kg Magnesiumstearat wird das Gemisch unter Anwendung eines Werkzeugs mit einem Durchmesser von 7 mm zu Tabletten verpreßt die auf beiden Seiten konvex sind, 10 mg Nifedipin enthalten und ein Gesamtgewicht von 0,09 g aufweisen. Die Tabletten werden mit 4 mg eines Überzugs auf der Basis von Hydroxypropylmethylcellulose versehen, der etwa 7,5 % gelbes Eisenoxidpigment und etwa 20 % Titandioxid enthält.

Nach der Bestimmung der In-vitro-Freisetzung (Ph.Hg. VII, "Drehflügelmethode"; 150 Umdrehungen/Minute; in 900 ml 0,1 N Salzsäure, Seite 473) werden 50 % des Wirkstoffs innerhalb von 45 bis 60 Minuten abgegeben. Gemäß einem In-vivo-Test wird der maximale Blutspiegel beim Hund gegen die 60. Minute festgestellt (W. Vater et al., Arzneimittelforschung 22, 1 (1972); H. Suzuki: J. Chromatogr. 341, 341 (1985)).

### Beispiel 8

12,0 kg Nifedipin werden in 96 kg Ethanol warm gelöst; die Lösung wird mit einer Lösung von 5,4 kg Polyvinylbutyral in 48 kg Ethanol vermischt. Die so erhaltene vereinigte Lösung wird auf ein gemisch von 54,0 kg mikrokristalliner Cellulose, 18,0 kg Milchzucker und 3,84 kg Croscarmellose in einem Wirbelschichtgranulierer (Glatt WSG 200) gesprüht. Der Sprühdurchsatz beträgt 2,0 kg/min, die Temperatur der zur Wirbelschichtzeugung dienenden Luft beträgt 45 °C. Nach Beendigung des Besprühens werden die Granalien an einem Schwing granulierer durch ein Sieb von etwa 0,7 mm gesiebt. Die Granalien werden in den Behälter des Wirbelschichtgranulierers zurückgeführt und dort mit einer Lösung von 1,20 kg Hydroxypropylcellulose und 24 kg Ethanol besprüht. Das gemisch wird in derselben Maschine getrocknet und gesiebt.

Den getrockneten Granalien werden in einer geeigneten Homogenisierungsvorrichtung 12,0 kg Croscarmellose, 1,2 g Talk und 0,36 kg Magnesiumstearat zugemischt. Das Gemisch wird mit einem konkaven Werkzeug (Durchmesser 8 mm) zu Tabletten mit einem Gesamtgewicht von 0,18 g verpreßt oder in Hartgelatinekapseln gefüllt. Die Tabletten werden mit einem üblichen Zuckerüberzug oder Filmüberzug versehen. Wenn das gemisch in Hartgelatinekapseln gefüllt wird, enthält die Kapselwand bzw. der Überzug vorteilhaft im orangeroten Bereich lichtundurchlässige Substanzen (z.B. Titandioxid, Eisenoxidpigment, rotes oder orangefarbiges Aluminiumpigment, usw.).

Nach einem In-vitro-Test werden 50 % des Wirkstoffs innerhalb von etwa 4 Stunden abgegeben (USA XXI oder Ph. Hg. VII, "Drehflügelmethode"; 150 Umdrehungen/min).

### Beispiel 9

500 g Nifedipin werden in 4000 g Ethanol warm gelöst. Zu der Lösung wird eine Lösung von 50 g Hydroxypropylcellulose, 175 g Polyvinylbutyral und 2000 g Ethanol zugegeben. Die vereinigte Lösung wird in einem Wirbelschicht granulierer auf ein gemisch von 2250 g mikrokristalliner Cellulose, 750 g Milchzucker und 160 g Croscarmellose gesprüht. Nach Beendigung des Besprühens werden die Granalien klumpenfrei gesiebt. Die Wirbelschicht granulierung wird durch Besprühen mit einer Lösung von 50 g Hydroxypropylcellulose und 1000 g Ethanol fortgesetzt. Die Granalien werden getrocknet und gesiebt. Nach Zugabe von 500 g Croscarmellose, 50 g Talk und 15 g Magnesiumstearat werden die Granalien zu linsenförmigen Tabletten gepreßt. Die Tabletten werden durch Besprühen mit einer Hydroxypropylmethylcelluloselösung, die lichtundurchlässige Pigmente, wie Titandioxid, Eisenoxidpigment, vorteilhaft rotes oder orangefarbiges Aluminiumpigment, enthält, mit einem Filmüberzug versehen. Nach einem In-vitro-Test werden 50 % des Wirkstoffs innerhalb von etwa 2 Stunden abgegeben (USP XXI oder Ph. Hg. VII, "Paddle"-Methode).

### Beispiel 10

110 g Nifedipin werden in 880 g Ethanol unter Erwärmen auf einem Wasserbad bei etwa 50 °C gelöst. Nach vollständiger Auflösung wird eine Lösung von 44 g Polyvinylbutyral in 720 g Ethanol zugegeben. In den Behälter eines Wirbelschichtgranulierers (Typ Glatt WSG 1) wird ein gemisch von 498 g mikrokristalliner Cellulose und 88 g Milchzucker eingewogen. Die Wirbelschicht wird bei einer Eingangslufttemperatur von 40 °C erzeugt, und die obige Lösung wird mit einem Durchsatz von 40 ml/min eingesprüht. Der Druck der eingesprühten Luft beträgt 0,5 bar.

Während des Besprühungsvorgangs wird die Lösung auf dem Wasserbad auf einer Temperatur von etwa 50 °C gehalten. Im Laufe dieses Vorgangs wird der Durchsatz der Lösung allmählich auf etwa 50 ml/min erhöht. Der Besprühungsvorgang dauert 40 min, die Temperatur der abgeführten Luft ist 22 bis 23 °C.

Nach Aufbringen des gesamten Flüssigkeitsvolumens wird eine Lösung von 16,5 g Hydroxypropylcellulose und 320 g Ethanol unter den obigen Bedingungen eingesprüht. Die so erhaltenen Granalien werden durch ein Sieb von etwa 0,9 mm gesiebt und danach in einer Wirbelschicht vorrichtung bis zu einer Ablufttemperatur von etwa 30 °C getrocknet. Nach Zumischung von 110 g Croscarmellose, 11 g Talk und 2,75 g Magnesiumstearat wird das so erhaltene Homogenisat mit Hilfe eines normalen, konkaven Werkzeugs mit einem Durchmesser von 8 mm zu Tabletten mit einem Gesamtgewicht von 0,16 g und einem Wirkstoffgehalt von 20 mg gepreßt.

Die Tabletten werden mit einem Filmüberzug versehen, der ein Titandioxidpigment und rotbraunes Eisenoxidpigment oder eine Mischung von "Sunset yellow" und ''Neococcin''-Aluminiumpigmenten enthält.

Die Wirkstoffabgabe der Tabletten wird nach der in USP XXI oder Ph. Hg. VII beschriebenen Methode (in 0,1 N Salzsäure, unter Anwendung von Körben) bestimmt. Die erhaltenen Ergebnisse sind in der nachstehenden Tabelle zusammengefaßt:

| Zeit (Stunden) | Freigesetztes Nifedipin (%) |
|---|---|
| 1 | 20 |
| 3 | 40 |
| 5 | 53 |

### Beispiel 11

50 g Nifedipin und 75 g Polyethylenglycol 6000 werden in 300 g Ethanol gelöst. Nach Auflösen werden 200 g einer 12,5 %igen Eudragit® RS-Lösung (siehe Beispiel 1) zugesetzt. In den Behälter eines Wirbelschicht granulierers (Typ Uniglatt) wird eine gemisch von 150 g Milchzucker und 150 g mikrokristalliner Cellulose eingewogen und mit der obigen, auf dem Wasserbad kontinuierlich bei 50 °C gehaltenen Lösung mit einem Durchsatz von 12 g/min besprüht. Die Temperatur der zur Wirbelschichterzeugung dienenden Luft beträgt 40 °C. Nach Beendigung des Besprühens werden die Granalien in derselben Vorrichtung getrocknet und danach durch ein Sieb mit einem Lochdurchmesser von etwa 0,9 mm gesiebt. Nach Zugabe von 50 g Crospovidon (s. Beispiel 1) wird das gemisch homogenisiert und in orange, rote oder braune lichtundurchlässige Hartgelatinekapseln (Größe 2) gefüllt. Das Gewicht der Füllung beträgt 0,2 g.

Die Wirkstoffabgabe der Kapseln wird nach der in USP XXI oder Ph.Hg. VII beschriebenen Methode bestimmt. Die erhaltenen Ergebnisse sind der nachstehenden Tabelle zu entnehmen:

| Zeit (Stunden) | Freigesetztes Nifedipin (%) |
|---|---|
| 1 | 22 |
| 2 | 32 |
| 3 | 41 |
| 4 | 48 |
| 5 | 54 |
| 6 | 60 |

## Patentansprüche

1. Nifedipin (4-(2'-Nitrophenyl)-2,6-dimethyl-3,5-dimethoxycarbonyl-1,4-dihydropyridin) enthaltende feste pharmazeutische Präparate zur therapeutischen Anwendung mit regulierter Wirkstoffabgabe, die mindestens ein pharmazeutisch inaktives Hydrophilisierungsmittel, das eine rasche Freisetzung des Nifedipins bewirkt, und mindestens ein pharmazeutisch inaktives Retardierungsmittel enthalten, welches eine verzögerte Freisetzung des Nifedipins bewirkt,
und die erhalten sind durch
(A) Aufbringen des Nifedipins sowie des Hydrophilisierungsmittels und des Retardierungsmittels in Lösung in einem organischen Lösungsmittel auf einen inerten Träger,
(B) Trocknen des behandelten Trägers,
(C) Vermischen mit üblichen Hilfsstoffen
und
(D) Tablettieren oder Abfüllen in Kapseln,
dadurch gekennzeichnet, daß
- das Nifedipin in kristalliner Form vorliegt,
- das Hydrophilisierungsmittel unter Hydroxypropylcellulose, Polyethylenglycolen oder einem grenzflächenaktiven Mittel ausgewählt ist,
- das Retardierungsmittel unter Polyvinylbutyral, Ethylcellulose, Polyvinylacetat oder einem Eudragit® ausgewählt ist und
- die Präparate dadurch erhältlich sind, daß Schritt A wie folgt durchgeführt wird:
(A1) Aufbringen einer Lösung des Nifedipins, des Hydrophilisierungsmittels und des Retardierungsmittels auf den Träger oder
(A2) Aufbringen einer Lösung des Nifedipins, des Retardierungsmittels sowie einer Teilmenge des Hydrophilisierungsmittels auf den Träger und abschließendes Aufbringen der Restmenge des Hydrophilisierungsmittels
oder
(A3) Aufbringen einer Lösung des Nifedipins und des Retardierungsmittels auf den Träger und anschließendes Aufbringen des Hydrophilisierungsmittels,
wobei die aufgebrachten Lösungen auf 1 Gew.-Teil Nifedipin 0,05 bis 1,5 Gew.-Teile Retardierungsmittel und insgesamt 0,1 bis 1,5 Gew.-Teile Hydrophilisierungsmittel enthalten.

2. Präparate nach Anspruch 1, dadurch erhältlich, daß zum Lösen des Nifedipins, des Hydrophilisierungsmittels und des Retardierungsmittels ein oder mehrere gleiche oder verschiedene organische Lösungsmittel verwendet werden.

3. Präparate nach Anspruch 2, dadurch erhältlich, daß als Lösungsmittel niedere Alkanole, vorzugsweise Ethanol oder Isopropanol, oder Aceton verwendet werden.

4. Präparate nach einem der Ansprüche 1 bis 3 mit verhältnismäßig rascher Wirkstoffabgabe, erhältlich durch Verwendung von 0,3 bis 1,5 Gew.-Teilen Hydrophilisierungsmittel und 0,05 bis 0,2 Gew.-Teilen Retardierungsmittel auf 1 Gew.-Teil Nifedipin.

5. Präparate nach einem der Ansprüche 1 bis 3 mit verzögerter Wirkstoffabgabe, erhältlich durch Verwendung von 0,1 bis 0,3 Gew.-Teilen Hydrophilisierungsmittel und 0,2 bis 1,5 Gew.-Teile Retardierungsmittel auf 1 Gew.-Teil Nifedipin.

6. Verfahren zur Herstellung der Präparate nach einem der Ansprüche 1 bis 5 durch
(A) Aufbringen des Nifedipins sowie des Hydrophilisierungsmittels und des Retardierungsmittels in Lösung in einem organischen Lösungsmittel auf einen inerten Träger,
(B) Trocknen des behandelten Trägers,
(C) Vermischen mit üblichen Hilfsstoffen
und
(D) Tablettieren oder Abfüllen in Kapseln,
dadurch gekennzeichnet, daß
- das Hydrophilisierungsmittel unter Hydroxypropylcellulose, Polyethylenglycolen oder einem grenzflächenaktiven Mittel ausgewählt wird,
- das Retardierungsmittel unter Polyvinylbutyral, Ethylcellulose, Polyvinylacetat oder einem Eudragit® ausgewählt wird und
- Schritt A wie folgt durchgeführt wird:
(A1) Aufbringen einer Lösung des Nifedipins, des Hydrophilisierungsmittels und des Retardierungsmittels auf den Träger oder
(A2) Aufbringen einer Lösung des Nifedipins, des Retardierungsmittels sowie einer Teilmenge des Hydrophilisierungsmittels auf den Träger und abschließendes Aufbringen der Restmenge des Hydrophilisierungsmittels
oder
(A3) Aufbringen einer Lösung des Nifedipins und des Retardierungsmittels auf den Träger und anschließendes Aufbringen des Hydrophilisierungsmittels,
wobei die aufgebrachten Lösungen auf 1 Gew.-Teil Nifedipin 0,05 bis 1,5 Gew.-Teile Retardierungsmittel und insgesamt 0,1 bis 1,5 Gew.-Teile Hydrophilisierungsmittel enthalten.

7. Verfahren nach Anspruch 6, dadurch gekennzeichnet, daß zur Herstellung von Präparaten mit verhältnismäßig rascher Wirkstoffabgabe 0,3 bis 1,5 Gew.-Teile Hydrophilisierungsmittel und 0,05 bis 0,02 Gew.-Teile Retardierungsmittel auf 1 Gew.-Teil Nifedipin verwendet werden.

8. Verfahren nach Anspruch 6, dadurch gekennzeichnet, daß zur Herstellung von Präparaten mit verzögerter Wirkstoffabgabe 0,1 bis 0,3 Gew.-Teile Hydrophilisierungsmittel und 0,2 bis 1,5 Gew.-Teile Retardierungsmittel auf 1 Gew.-Teil Nifedipin verwendet werden.

9. Verfahren nach einem der Ansprüche 6 bis 8, dadurch gekennzeichnet, daß zum Lösen des Nifedipins, des Hydrophilisierungsmittels und des Retardierungsmittels ein oder mehrere gleiche oder verschiedene organische Lösungsmittel verwendet werden.

10. Verfahren nach Anspruch 9, dadurch gekennzeichnet, daß als Lösungsmittel niedere Alkanole, vorzugsweise Ethanol oder Propanol, oder Aceton verwendet werden.

11. Verfahren nach einem der Ansprüche 6 bis 10, dadurch gekennzeichnet, daß als Hydrophilisierungsmittel Hydroxypropylcellulose und als Retardierungsmittel Polyvinylbutyral verwendet werden und die Komponenten Nifedipin, Hydroxypropylcellulose und Polyvinylbutyral durch Sprühen entsprechender Lösungen auf den Träger aufgebracht werden.

12. Nifedipin-Kombinationspräparate, gekennzeichnet durch mindestens ein Präparat mit verhältnismäßig rascher Wirkstoffabgabe, insbesondere nach Anspruch 4, sowie mindestens ein Präparat mit verzögerter Wirkstoffabgabe, insbesondere nach Anspruch 5, wobei die beiden Präparatarten vorzugsweise gemeinsam in Kapseln oder zusammen in Tabletten bzw. Dragees verpreßt vorliegen.

13. Nifedipin-Kombinationspräparate nach Anspruch 12, dadurch gekennzeichnet. daß die Tabletten bzw. Dragees eine äußere Schicht aus einem Präparat mit verhältnismäßig rascher Wirkstoffabgabe, insbesondere nach Anspruch 4, und einen Kern aus einem Präparat mit verzögerter Wirkstoffabgabe, insbesondere nach Anspruch 5, aufweisen.

## Claims

1. Nifedipine (4-(2'-nitrophenyl)-2,6-dimethyl-3,5-dimethoxycarbonyl-1,4-dihydropyridine) containing solid pharmaceutical preparations for therapeutic use with controlled active ingredient release, which contain at least one pharmaceutically inactive hydrophilising agent effecting rapid release of nifedipine, and at least one pharmaceutically inactive retardant effecting delayed release of nifedipine,
and which are obtained by
(A) applying the nifedipine and the hydrophilising agent and retardant in solution in an organic solvent to an inert support,
(B) drying the treated support,
(C) mixing with conventional additives
and
(D) tabletting or packing in capsules,
characterised in that
- the nifedipine is in crystalline form,
- the hydrophilising agent is selected from hydroxypropyl cellulose, polyethylene glycols or an interface-active agent,
- the retardant is selected from polyvinyl butyral, ethyl cellulose, polyvinyl acetate or an Eudragit®
and
- the preparations are obtainable by performing step (A) as follows:
(A1) application of a solution of nifedipine, hydrophilising agent and retardant to the support
or
(A2) application of a solution of nifedipine, retardant and some of the hydrophilising agent to the support followed by application of the remainder of the hydrophilising agent
or
(A3) application of a solution of nifedipine and retardant to the support followed by application of the hydrophilising agent,
the applied solutions containing, to 1 part by weight of nifedipine, 0.05 to 1.5 parts by weight of retardant and a total of 0.1 to 1.5 parts by weight of hydrophilising agent.

2. Preparations according to claim 1, characterised in that one or more identical or different organic solvents are used to dissolve the nifedipine, hydrophilising agent and retardant.

3. Preparations according to claim 2, obtainable by the use of lower alkanols, preferably ethanol or isopropanol, or acetone, as solvent.

4. Preparations according to any one of claims 1 to 3 with a relatively rapid active ingredient release, obtainable by using 0.3 to 1.5 parts by weight of hydrophilising agent and 0.05 to 0.2 parts by weight of retardant to 1 part by weight of nifedipine.

5. Preparations according to any one of claims 1 to 3 with delayed active ingredient release, obtainable by using 0.1 to 0.3 parts by weight of hydrophilising agent and 0.2 to 1.5 parts by weight of retardant to 1 part by weight of nifedipine.

6. A process for the production of preparations according to any one of claims 1 to 5 by
(A) applying the nifedipine and the hydrophilising agent and retardant in solution in an organic solvent to an inert support,
(B) drying the treated support,
(C) mixing with conventional additives
and
(D) tabletting or packing in capsules,
characterised in that
- the hydrophilising agent is selected from hydroxypropyl cellulose, polyethylene glycols or an interface-active agent,
- the retardant is selected from polyvinyl butyral, ethyl cellulose, polyvinyl acetate or an Eudragit®
and
- step A is performed as follows
(A1) application of a solution of nifedipine, hydrophilising agent and retardant to the support
or
(A2) application of a solution of nifedipine, retardant and some of the hydrophilising agent to the support followed by application of the remainder of the hydrophilising agent
or
(A3) application of a solution of nifedipine and retardant to the support followed by application of the hydrophilising agent,
the applied solutions containing, to 1 part by weight of nifedipine, 0.05 to 1.5 parts by weight of retardant and a total of 0.1 to 1.5 parts by weight of hydrophilising agent.

7. A process according to claim 6, characterised in that 0.3 to 1.5 parts by weight of hydrophilising agent and 0.05 to 0.02 parts by weight of retardant are used to 1 part by weight of nifedipine for the production of preparations having relatively rapid active ingredient release.

8. A process according to claim 6, characterised in that 0.1 to 0.3 parts by weight of hydrophilising agent and 0.2 to 1.5 parts by weight of retardant are used to 1 part by weight of nifedipine for the production of preparations having delayed active ingredient release.

9. A process according to any one of claim 6 to 8, characterised in that one or more identical or different organic solvents are used to dissolve the nifedipine, hydrophilising agent and retardant.

10. A process according to claim 9, characterised in that lower alkanols, preferably ethanol or isopropanol, or acetone, are used as solvent.

11. A process according to any one of claims 6 to 10, characterised in that hydroxypropyl cellulose is used as hydrophilising agent and polyvinyl butyral is used as retardant and the components nifedipine, hydroxypropyl cellulose and polyvinyl butyral are applied to the support by spraying corresponding solutions.

12. Nifedipine combination preparations characterised by at least one preparation having a relatively rapid active ingredient release, more particularly according to claim 4, and at least one preparation having a delayed active ingredient release, more particularly according to claim 5, the two types of preparation preferably being present jointly in capsules or compressed together in tablets or dragées.

13. Nifedipine combination preparations according to claim 12, characterised in that the tablets or dragées have an outer layer consisting of a preparation with a relatively rapid active ingredient release, more particularly according to claim 4, and a core of a preparation having a delayed active ingredient release, more particularly according to claim 5.

## Revendications

1. Produits pharmaceutiques solides contenant de la nifédipine (4-(2'-nitrophényl)-2,6-diméthyl-3,5-diméthoxycarbonyl-1,4-dihydropyridine), pour usage thérapeutique, à libération modulée du principe actif, qui contiennent au moins un agent hydrophilisant pharmaceutiquement inactif qui induit une libération rapide de la nifédipine et au moins un agent retard pharmaceutiquement inactif qui induit une libération retardée de la nifédipine, et qui sont obtenus par
(A) dépôt de la nifédipine ainsi que de l'agent hydrophilisant et de l'agent retard en solution dans un solvant organique sur un support inerte,
(B) séchage du support traité,
(C) mélange avec des adjuvants usuels,
et
(D) production de comprimés ou introduction dans des gélules,
caractérisés en ce que
- la nifédipine se présente sous une forme cristalline,
- l'agent hydrophilisant est sélectionné parmi l'hydroxypropylcellulose, les polyéthylèneglycols ou un agent tensioactif,
- l'agent retard est sélectionné parmi le poly(butyral de vinyle), l'éthylcellulose, le poly(acétate de vinyle) ou un Eudragit®
et
- les produits peuvent être obtenus par le fait que l'étape A est effectuée comme suit :
(A1) dépôt d'une solution de nifédipine, d'agent hydrophilisant et d'agent retard sur le support
ou
(A2) dépôt d'une solution de nifédipine, d'agent retard ainsi qu'une quantité partielle d'agent hydrophilisant sur le support et dépôt subséquent de la quantité résiduelle d'agent hydrophilisant
ou
(A3) dépôt d'une solution de nifédipine et d'agent retard sur le support et dépôt subséquent de l'agent hydrophilisant,
les solutions déposées contenant, pour 1 partie en poids de nifédipine, 0,05 à 1,5 partie en poids d'agent retard et au total 0,1 à 1,5 partie en poids d'agent hydrophilisant.

2. Produits selon la revendication 1, qu'il est possible d'obtenir par le fait qu'on utilise un ou plusieurs solvants organiques identiques ou différents pour dissoudre la nifédipine, l'agent hydrophilisant et l'agent retard.

3. Produits selon la revendication 2, qu'il est possible d'obtenir par le fait qu'on utilise comme solvant des alcanols inférieurs, de préférence éthanol ou isopropanol, ou de l'acétone.

4. Produits selon une des revendications 1 à 3, à libération relativement rapide du principe actif, qu'il est possible d'obtenir par utilisation de 0,3 à 1,5 partie en poids d'agent hydrophilisant et de 0,05 à 0,2 partie en poids d'agent retard pour 1 partie en poids de nifédipine.

5. Produits selon une des revendications 1 à 3, à libération retardée du principe actif, qu'il est possible d'obtenir par utilisation de 0,1 à 0,3 partie en poids d'agent hydrophilisant et de 0,2 à 1,5 partie en poids d'agent retard pour 1 partie en poids de nifédipine.

6. Procédé de préparation des produits selon une des revendications 1 à 5, par
(A) dépôt de la nifédipine ainsi que de l'agent hydrophilisant et de l'agent retard en solution dans un solvant organique sur un support inerte,
(B) séchage du support traité,
(C) mélange avec des adjuvants usuels
et
(D) production de comprimés ou introduction dans des gélules,
caractérisé en ce que
- l'agent hydrophilisant est sélectionné parmi l'hydroxypropylcellulose, les polyéthylèneglycols ou un agent tensioactif,
- l'agent retard est sélectionné parmi le poly(butyral de vinyle), l'éthylcellulose, le poly(acétate de vinyle) ou un Eudragit®
et
- l'étape A est effectuée comme suit :
(A1) dépôt d'une solution de nifédipine, d'agent hydrophilisant et d'agent retard sur le support
ou
(A2) dépôt d'une solution de nifédipine, d'agent retard ainsi que d'une quantité partielle d'agent hydrophilisant sur le support et dépôt subséquent de la quantité résiduelle d'agent hydrophilisant
ou
(A3) dépôt d'une solution de nifédipine et d'agent retard sur le support et dépôt subséquent de l'agent hydrophilisant,
les solutions déposées contenant, pour 1 partie en poids de nifédipine, 0,05 à 1,5 partie en poids d'agent retard et au total 0,1 à 1,5 partie en poids d'agent hydrophilisant.

7. Procédé selon la revendication 6, caractérisé en ce qu'on utilise pour préparer les produits à libération relativement rapide du principe actif 0,3 à 1,5 partie en poids d'agent hydrophilisant et 0,05 à 0,02 partie en poids d'agent retard pour 1 partie en poids de nifédipine.

8. Procédé selon la revendication 6, caractérisé en ce qu'on utilise pour préparer les produits à libération retardée du principe actif 0,1 à 0,3 partie en poids d'agent hydrophilisant et 0,2 à 1,5 partie en poids d'agent retard pour 1 partie en poids de nifédipine.

9. Procédé selon une des revendications 6 à 8, caractérisé en ce qu'on utilise un ou plusieurs solvants organiques identiques ou différents pour dissoudre la nifédipine, l'agent hydrophilisant et l'agent retard.

10. Procédé selon la revendication 9, caractérisé en ce qu'on utilise comme solvant des alcanols inférieurs, de préférence éthanol ou propanol, ou de l'acétone.

11. Procédé selon une des revendications 6 à 10, caractérisé en ce qu'on utilise de l'hydroxypropylcellulose comme agent hydrophilisant et du poly(butyral de vinyle) comme agent retard et en ce qu'on dépose les composants nifédipine, hydroxypropylcellulose et poly(butyral de vinyle) sur le support par pulvérisation de solutions correspondantes.

12. Associations de nifédipine, caractérisées par la présence d'au moins un produit à libération relativement rapide du principe actif, notamment selon la revendication 4, ainsi que d'au moins un produit à libération retardée du principe actif, notamment selon la revendication 5, les deux types de produits étant de préférence réunis dans des gélules ou compressés ensemble en comprimés ou comprimés dragéifiés.

13. Associations de nifédipine selon la revendication 12, caractérisées en ce que les comprimés ou comprimés dragéifiés présentent une couche externe constituée par un produit à libération relativement rapide du principe actif, notamment selon la revendication 4, et un noyau constitué par un produit à libération retardée du principe actif, notamment selon la revendication 5.
